# EUROPEAN PATENT SPECIFICATION

(11) **EP 2 935 599 B1**
(45) Date of publication and mention of the grant of the patent: **22.11.2017**
(21) Application number: 13814542.0
(22) Date of filing: 19.12.2013
(51) Int. Cl.: C12P 7/62, C12N 9/00, C12N 9/10, C12N 9/02, C12N 9/88, C12N 9/04, C12N 9/20, C08G 63/06

(54) **PREPARATION OF LONG-CHAIN LENGTH POLY(HYDROXYFATTY ACIDS)**
HERSTELLUNG VON POLY(HYDROXYFETTSÄUREN) LANGKETTIGER LÄNGE
PRÉPARATION DE POLY(HYDROXYFATTY ACIDES) À CHAÎNE LONGUE

(30) Priority: 21.12.2012 EP 12306663
(43) Date of publication of application: 28.10.2015
(73) Proprietor: Carbios, 63360 Saint-Beauzire (FR)
(72) Inventor: BOISART, Cédric, F-63360 Gerzat (FR); MARTY, Alain, F-31570 Saint-Pierre de Lages (FR)
(74) Representative: Cabinet Becker et Associés
(86) International application number: PCT/EP2013/077522
(87) International publication number: WO 2014/096276

(56) References cited:
- EP-A1- 2 418 277
- WO-A1-01/09364
- WO-A1-2007/017270
- WO-A1-2008/135065
- WO-A1-2012/071657
- WO-A2-2007/029213
- WO-A2-2009/156950
- LIU Q ET AL: "Biosynthesis of poly(3-hydroxydecanoate) and 3-hydroxydodecanoate dominating polyhydroxyalkanoates by beta-oxidation pathway inhibited Pseudomonas putida", METABOLIC ENGINEERING, ACADEMIC PRESS, US, vol. 13, no. 1, 1 January 2011 (2011-01-01), pages 11-17, XP027575822, ISSN: 1096-7176 [retrieved on 2010-10-27]
- AKHILESH KUMAR SINGH ET AL: "Exploitation of inexpensive substrates for production of a novel SCLâ LCL-PHA co-polymer by Pseudomonas aeruginosa MTCC 7925", JOURNAL OF INDUSTRIAL MICROBIOLOGY & BIOTECHNOLOGY ; OFFICIAL JOURNAL OF THE SOCIETY FOR INDUSTRIAL MICROBIOLOGY, SPRINGER, BERLIN, DE, vol. 36, no. 3, 4 December 2008 (2008-12-04), pages 347-354, XP019665566, ISSN: 1476-5535
- SHAO-PING OUYANG ET AL: "Production of Polyhydroxyalkanoates with High 3-Hydroxydodecanoate Monomer Content by fadB and fadA Knockout Mutant of Pseudomonas putida KT2442", BIOMACROMOLECULES, vol. 8, no. 8, 1 August 2007 (2007-08-01), pages 2504-2511, XP055019220, ISSN: 1525-7797, DOI: 10.1021/bm0702307
- SURIYAMONGKOL ET AL: "Biotechnological approaches for the production of polyhydroxyalkanoates in microorganisms and plants - A review", BIOTECHNOLOGY ADVANCES, ELSEVIER PUBLISHING, BARKING, GB, vol. 25, no. 2, 26 January 2007 (2007-01-26), pages 148-175, XP005862313, ISSN: 0734-9750, DOI: 10.1016/J.BIOTECHADV.2006.11.007

## Description

The present invention concerns a method for the preparation of long chain length poly(hydroxyfatty acids) from vegetable oils.

### Background of the invention

Polyhydroxyalkanoates (PHAs) are polyesters formed by the polymerization of hydroxylated alkanoates, in particular hydroxylated fatty acids. PHAs prepared from hydroxylated fatty acids are named polyhydroxyfatty acids (PHFAs). PHAs and PHFAs can be used for a wide variety of commodity plastics applications. Unlike conventional plastics, PHAs and PHFAs are biodegradable in nature, thus they are an environment-friendly class of polymers. PHAs and PHFAs can for instance be used as novel bioresorbable medical materials.
PHAs can be synthesized via polymerization of hydroxyacids. PHAs are naturally synthesized by a wide range of different Gram-positive and Gram-negative bacteria, as well as by some Archaea.

Both chemical and enzymatic approaches have been explored to synthesize polyesters from hydroxyacid monomers. Chemical synthetic methods often require harsh reaction conditions and metal catalysts that are difficult to remove subsequent to polymerization. Compared to chemical preparation, enzyme-catalyzed polymerizations can be performed under mild reaction conditions, using proteins that are metal-free and that have high enantio- and regio-selectivities.

Polymerization of PHFAs from fatty acids proceeds in two consecutive steps:
- oxidation of fatty acids into hydroxylated fatty acids, and
- polymerization of the obtained hydroxylated fatty acids by reaction between hydroxyl and carboxyl groups of hydroxylated fatty acids.

Preparation of PHFAs from short chain (C3-C5) and medium chain (C6-C14) length fatty acids with microorganisms has been widely described in literature. Conversely, the preparation of PHFAs from long chain length (at least C16) fatty acids with microorganisms has been the object of very few disclosures. Actually, the native enzymes of microorganisms that contribute to at least one of the above described steps of oxidation and polymerization are appropriate for the preparation of polyhydroxyfatty acids from short and medium chain length hydroxyfatty acids.

In addition, most described methods for the preparation of PHFAs use fatty acids as starting material. The nature of the fatty acids used as starting material is chosen depending on the desired PHFA to form. Nevertheless, fatty acids are not naturally available as isolated pure fatty acids, but are mainly present in the form of oils. Oils typically comprise mixtures of triglycerides of different chain length, said triglycerides being formed by the esterification of glycerol and fatty acids. Said methods thus require, before implementation of the oxidation and polymerization steps, at least one preliminary step of isolating and/or purifying the desired fatty acid(s). In addition, one of the inconvenient of enzyme-catalyzed preparation of PHAs is the high cost of bacterial fermentation. There is thus an interest in developing enzyme-catalyzed preparation of PHAs involving inexpensive raw materials as substrates, and requiring few purification and/or isolation steps. In particular, it would be highly interesting to be able to synthesize PHFAs directly from natural sources of fatty acids, such as vegetable oils.

In this context, the Applicant developed a new method, preferably enzyme-catalyzed method, for the preparation of long-chain PHFAs directly from vegetable oils.

### Summary of the invention

An object of the present invention is a method for the preparation of long-chain PHFAs, according to claim 1.

### Detailed description of the invention

The method of the invention comprises the following steps:
a) Obtaining of long chain length fatty acids comprising at least 16 carbon atoms from oil,
b) Oxidation of long chain length fatty acids to form hydroxy(fatty acids) or derivatives thereof such as CoA thioesters thereof,
c) Polymerization of the hydroxy(fatty acids) or derivatives thereof obtained in step b to form at least one PHFA, and
d) Recovery of the at least one PHFA, wherein step b) is a ω oxidation, performed in a recombinant microorganism comprising at least one appropriate cytochrome P450 enzyme.

### Step a: obtaining of long chain length fatty acids from oil

The oil may be any oil comprising triglycerides with long fatty chains comprising at least 16 carbon atoms, preferably from 16 to 24 carbon atoms, more preferably from 16 to 20 carbon atoms or from 18 to 24 carbon atoms, in particular 16, 18 or 20 carbon atoms, or mixtures thereof. Preferably, the oil comprises at least 25%, preferably at least 50%, preferably at least 70%, preferably at least 80% of mono-unsaturated or polyunsaturated fatty chains, preferably of mono-unsaturated fatty chains.

The long chain length fatty acids obtained in step a may be in particular oleic and/or linoleic acids.
In a preferred embodiment, the oil is a vegetable oil, or a mixture of such oils. Among oils that can be used in the present invention, one can cite rapeseed oil (also named canola oil), sunflower oil, peanut oil (also named groundnut oil), corn oil, olive oil, cotton seed oil, sesame oil, palm oil, soybean oil, mustard oil, and any mixture thereof. In an embodiment of the invention, the oil is selected in the group consisting of: rapeseed oil (also named canola oil), sunflower oil, peanut oil (also named groundnut oil), corn oil, olive oil, cotton seed oil, sesame oil, and any mixture thereof.
In a preferred embodiment, the oil is selected in the group consisting of: rapeseed oil (also named canola oil), sunflower oil, peanut oil (also named groundnut oil), corn oil, olive oil, and any mixture thereof.
In a highly preferred embodiment, the oil is selected in the group consisting of: rapeseed oil (also named canola oil), sunflower oil, and any mixture thereof. In an embodiment, the oil is rapeseed oil. In another embodiment, the oil is sunflower oil.

Preferably, step a involves transformation of at least some of the triglycerides comprised in the oil into long chain length fatty acids and glycerol. In particular, step a involves lipolysis of at least some of the triglycerides comprised in the oil to obtain long chain length fatty acids and glycerol, preferably with a lipase. Step a may also be performed by alcoholysis or hydrolysis.

In the present invention, the term "long chain length fatty acid" refers to a carboxylic acid with a long aliphatic tail (or chain), which is either saturated or unsaturated and comprises at least 16 carbon atoms, preferably from 16 to 24 carbon atoms, more preferably from 16 to 20 carbon atoms, in particular they comprise 16, 18 and/or 20 carbon atoms. Among long chain length fatty acids in the scope of the present invention can be cited for instance oleic acid, linoleic acid, and any mixture thereof. In an embodiment, the long chain length fatty acids of the invention are mono- or polyunsaturated long chain length fatty acids, preferably mono-unsaturated long chain length fatty acids. The unsaturations are highly preferably double C=C bonds.

In an embodiment, the method of the invention may comprise, after step a, preferably between step a and step b, a further step a' consisting of separation of at least one fatty acid from the other components obtained after step a, in particular glycerol.

The method of the invention may also comprise, after step a, preferably between step a and step b, for instance after step a', a further step a" consisting of separation of the fatty acids depending on their chain length. For instance, step a" may consist in separating the long chain length fatty acids from the other fatty acids, and optionally from glycerol.

In a preferred embodiment, the glycerol that is obtained after step a, a' or a" is further used in step b as carbon source for the recombinant microorganism.

In an embodiment, the method of the invention does not comprise step a. In said embodiment, step b is preferably performed with long-chain fatty acids, which were previously obtained from an oil as defined above.

### Step b: Oxidation of fatty acids to form hydroxylated fatty acids

The hydroxylation of fatty acids is performed in ω position of the fatty chain. Preferably, only one position of the fatty acid is hydroxylated in step b. Step b is performed by contacting the fatty acids with at least one enzyme appropriate for oxidizing the desired fatty acids in the desired position. According to a specific embodiment, step b involves a microorganism, preferably a recombinant microorganism.
In the present invention, the term "microorganism" designates a bacterium, yeast or fungus. Preferably, the microorganism is a microorganism of a genus selected among *Cupriavidus, Escherichia, Pseudomonas, Yarrowia, Aeromonas, Candida, Burkholderia, Saccharomyces* and *Bacillus.* More preferably, the microorganism is selected among *Cupriavidus necator, Escherichia coli, Pseudomonas aeruginosa, Pseudomonas putida, Pseudomonas entomophila, Pseudomonas oleovorans, Yarrowia lipolytica, Aeromonas hydrophila, Candida tropicalis, Candida antarctica, Burkholderia xenovorans, Burkholderia mallei, Burkholderia pseudomallei, Saccharomyces cerevisiae, Bacillus subtilis* and *Bacillus megaterium.*
A recombinant microorganism is a microorganism as defined above that has been modified by genetic engineering.
Alternatively, step b may be performed *ex vivo.*

### β oxidation

In an embodiment of the disclosure, the oxidation of the long chain length fatty acids is a β oxidation, meaning that, for a fatty acid comprising n carbon atoms, the hydroxyl OH group is linked to the carbon atom number 3. An example of hydroxylated fatty acid obtained by β oxidation is the compound of formula

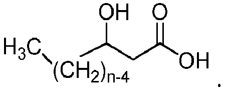

In the embodiment wherein the oxidation of the long chain length fatty acids is a β oxidation, the method further comprises, before step b, a step a''' consisting of activation of the fatty acids as to form CoA thioesters of the fatty acids. The activation of fatty acids into CoA thioesters may be performed with a CoA transferase, for instance with acetylCoA as co-substrate, or with a CoA synthetase. Preferably, the activation of fatty acids into CoA thioesters is performed with a CoA synthetase, such as the CoA synthetase encoded by the fadD gene of *Escherichia coli.*

The β oxidation of the long chain length fatty acids preferably involves a recombinant microorganism. Alternatively, the β oxidation may be performed by contacting the long chain length fatty acids or derivatives thereof such as CoA thioesters thereof with the appropriate enzymes, and optionally at least one appropriate cofactor (such as NAD⁺, FAD or HSCoA) and/or further substrate, *ex vivo.* For instance, the enzymes may be immobilized on a support such as polymer beads.

The microorganism used may use at least one carbon source, preferably different from the long chain length fatty acids. In a preferred embodiment, the glycerol obtained in step a, a' or a" is used as carbon source by the microorganism.
The term "carbon source" designates any source of carbon that can be used to support the normal growth of a microorganism, such as hexoses (for instance glucose, galactose or lactose), pentoses, monosaccharides, disaccharides (for instance sucrose, cellobiose or maltose), oligosaccharides, molasses, starch or its derivatives, hemicelluloses, glycerol or any combination thereof. A preferred carbon source is glycerol.

The minimal requirement for the microorganism to form β hydroxylated fatty acids or a derivative thereof, preferably a CoA thioester thereof, from long chain length fatty acids is the presence in the microorganism of appropriate enzymes to transform long chain length fatty acids into β hydroxylated fatty acids or derivatives thereof, such as CoA thioesters thereof. In particular, the microorganism may comprise at least one enzyme among: a CoA transferase or a CoA synthetase, such as the CoA synthetase encoded by the fadD gene of *Escherichia coli,* an AcylCoA oxidase and a 2-enoylCoA hydratase. Preferably, the microorganism comprises a CoA transferase or a CoA synthetase, such as the CoA synthetase encoded by the fadD gene of *Escherichia coli,* an AcylCoA oxidase (or dehydrogenase) and a 2-enoylCoA hydratase. Preferably, the AcylCoA oxidase (or dehydrogenase) is specific for long chain length fatty acids.
Each of the necessary enzymes as described above may be either naturally present in the microorganism or specifically provided to the organism by engineering.

Provision of the necessary enzymes, and optimization of their efficacy, may be performed by addition, enhancement, silencing, disruption and/or deletion of genes in the microorganism.
In particular, the genes coding for the enzymes implied in the degradation of CoA thioesters of β hydroxylated fatty acids, such as 3-hydroxyacyl-CoA dehydrogenase and/or 3-ketoacyl CoA thiolase, may be silenced or deleted in the microorganism.
The genes coding for the enzymes implied in the formation of CoA thioesters of β hydroxylated fatty acids, such as CoA synthetase or CoA transferase, acyl CoA oxidase (or dehydrogenase), and/or enoyl CoA hydratase may be added or enhanced in the microorganism.

In an embodiment, at least one gene among the genes for acyl CoA synthetase (fadD), the genes for acyl CoA oxidase (or dehydrogenase) (fadE or yafH) and the genes for enoyl CoA hydratase (fadB) of *Escherichia coli* may be enhanced.
In an embodiment, at least one gene selected from the genes for 3-hydroxyacyl CoA dehydrogenase (fadB) and the genes for 3-ketoacyl CoA thiolase (fadA), of *Escherichia coli* may be silenced or deleted.

One of ordinary skill in the art is able to adapt the genes described above for *Escherichia coli* to any other microorganism of interest.

The term "addition" of a gene coding for an enzyme designates the introduction of a new gene or an extra copy of an existing gene into the microorganism. For instance, a method for introducing DNA in *Escherichia coli* is electroporation. The addition may be performed by any appropriate vector, such as a plasmid or a phage. Alternatively, the gene may be added to the microorganism by integration into its chromosome(s).
The term "enhancement" of a gene coding for an enzyme designates that the encoded enzyme is expressed or overexpressed, meaning that the transcription and/or translation of the gene is increased compared to a non-recombinant microorganism, leading to an increased amount of enzyme in the cell. Preferably, the enzyme expression is increased of at least 20%, at least 50%, at least 70%, at least 90% or around 100%. One of ordinary skill in the art knows different ways to increase the expression of a gene. For instance, the gene may be expressed using promoter with different strength, which may be inducible. These promoters may be homologous or heterologous. One of ordinary skill in the art knows which promoters are the most appropriate. Examples of promoters are Ptrc, Ptac, Plac, or cI.
The terms "silencing" and "disruption" of a gene coding for an enzyme designate that the encoded enzyme is less expressed, meaning that the transcription and/or translation of the gene is lowered compared to a non-recombinant microorganism, leading to a lower amount of enzyme in the cell. Preferably, the enzyme expression is lowered of at least 20%, at least 50%, at least 70%, at least 90% or around 100%. One of ordinary skill in the art knows different ways to lower the expression of a gene.
The term "deletion" of a gene coding for an enzyme designates that the gene is removed from the microorganism chromosome(s). One of ordinary skill in the art knows different ways to delete a gene.

### ω oxidation

The oxidation of the long chain length fatty acids of the method of the invention is a ω oxidation, meaning that, for a long chain length fatty acid comprising n carbon atoms, the hydroxyl OH group is linked to the carbon atom number n. An example of hydroxylated fatty acid obtained by ω oxidation is the compound of formula

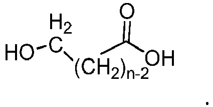

The ω oxidation of the long chain length fatty acids preferably involves a recombinant microorganism. Alternatively, the ω oxidation may be performed by contacting the long chain length fatty acids with the appropriate enzymes, and optionally at least one appropriate cofactor (such as NAD⁺, FAD or HSCoA) and/or further substrate, *ex vivo.* For instance, the enzymes may be immobilized on a support such as polymer beads.

The minimal requirement for the microorganism to form ω hydroxylated fatty acids from long chain length fatty acids is the presence in the microorganism of appropriate enzymes to transform fatty acids into ω hydroxylated fatty acids. In particular, the microorganism comprises at least one appropriate cytochrome P450 enzyme. Examples of appropriate cytochrome P450 enzymes are CYP52A from *Candida tropicalis,* CYP94A1 from *Vicia sativa,* CYP 94A5 from *Nicotiana tabacum,* CYP78A1 from *Zea mays,* CYP 86A1 and CYP86A8 from *Arabidopsis thaliana,* CYP 92B1 from *Petunia hybrida,* CYP102A1 (BM-3) mutant F87 from *Bacillus megaterium* and CYP 4 family from mammal and insect.
When step b is an in-chain oxidation, step b may involve a microorganism comprising at least one appropriate cytochrome P450 enzyme. Examples of appropriate cytochrome P450 enzymes are CYP81B1 from *Helianthus tuberosus* (ω-1 to ω-5 hydroxylation), CYP790C1 from *Helianthus tuberosus* (ω-1 and ω-2 hydroxylation), CYP152B1 *from Sphingomonas paucimobilis* (α-hydroxylation), CYP2E1 and 4A1 from human liver (ω-1 hydroxylation); P450BSβ from *Bacillus subtilis* (α and β-hydroxylation), and CYP102A1 (BM-3) from *Bacillus megaterium* (ω-1, ω-2 and ω-3 hydroxylation).

In addition to naturally occurring enzymes, modified enzymes may be added into the microorganism genome. For example, modified versions of cytochrome P450s may be obtained with improved ability to oxidize fatty acids of different lengths (for example C16, C17, C18, C19, C20, C21, C22, C23, C24) or different degrees of saturation.

### Step c: Polymerization of the hydroxylated fatty acids, or derivatives thereof such as CoA thioesters thereof, to form at least one PHFA

The step of polymerization may be performed in any conditions appropriate for obtaining the desired polymer or mixture of polymers. The polymerization of the hydroxylated fatty acids consists in the chemical reactions of at least one hydroxyl group present on a hydroxylated fatty acid, on a derivative thereof such as a CoA ester, or on a polymer that has already started to form, with at least one carboxyl COOH group or CoA ester group present on a hydroxylated fatty acid, on a derivative thereof such as a CoA ester, or on a polymer that has already started to form.

One of ordinary skill in the art is able to adapt the conditions to obtain the desired polymer(s), in particular in terms of number of monomers. In the present invention, the term "monomer" refers to the hydroxylated fatty acids, or the CoA thioesters thereof, that are polymerized to form the PHFAs.

The polymerization may be performed in a microorganism. The polymerization may be performed for instance in intracellular conditions, in particular with a PHA synthase. In these conditions, the PHFAs are produced in the cell. The polymerization is advantageously performed with a PHA synthase when step b of the method is a β oxidation. When step b of the method is a ω oxidation, the polymerization may also be performed with a PHA synthase. In this case, the method of the invention advantageously comprises, between step b and step c, a further step b' of activation of the hydroxylated fatty acids obtained in step b into the corresponding CoA thioesters. For instance, step b' can be performed with a CoA synthetase.

The PHA synthase may be naturally present in the native microorganism, or provided to the microorganism. Preferably, the PHA synthase is provided to the microorganism by engineering, in particular by addition or enhancement of appropriate genes in the microorganism. The method may also require silencing, disruption and/or deletion of some genes in the microorganism.

PHA synthase refers to an enzyme that polymerizes long chain length hydroxyfatty acids or derivatives thereof, such as CoA thioesters thereof, monomer units to form polymer. Examples of PHA synthases include the PHA synthase encoded by the phaC1 gene from *Pseudomonas oleovorans* or *Pseudomonas aeruginosa,* the synthase from *Cupriavidus necator* and the synthase from *Thiocapsa pfennigii* encoded by phaE and phaC.
In particular, the PHA synthase is an evolved PHA synthase that has been modified so as to be efficient at polymerizing long chain length (*ie* with at least 16 carbon atoms) hydroxylated fatty acids.

When steps b and c both involve microorganisms as defined above, the microorganism used in step c is preferably the same as the one used in step b.

Alternatively, the polymerization may be performed in extracellular conditions, in particular with a lipase. One of ordinary skill in the art is able to adapt the conditions, in particular the thermodynamical conditions, for the polymerization to obtain the desired polymer(s). For instance, the polymerization may be performed by contacting the hydroxylated fatty acids with an immobilized enzyme catalyst. One example of an immobilized enzyme catalyst that can be used is immobilized *Candida antarctica* Lipase B (CALB). Novozym 435 is an example of immobilized *Candida antarctica* Lipase B, where the immobilization support consists of macroporous polymethylmethacrylate beads.
When step c is performed in extracellular conditions, the method of the invention preferably comprises, preferably between step b and step c, an additional step b" consisting in transforming the CoA thioesters of the long chain length hydroxylated fatty acids obtained in step b into the corresponding long chain length hydroxylated fatty acids. Step b" may be performed with a CoA transferase, for instance with acetylCoA as co-substrate, or with a thioesterase.

The PHFA(s) obtained by the method of the invention are further objects of the invention, either as mixture or after separation such as separation depending on molecular weight or monomer nature.

Each of the PHFA obtained by the method of the invention may be a homopolymer or a copolymer of hydroxyfatty acids. The PHFA preferably comprises more than 50%, preferably more than 60%, preferably more than 70%, preferably more than 80%, preferably more than 90%, preferably more than 95%, in particular around 100%, of hydroxylated fatty acids monomers comprising at least 16 carbon atoms, preferably at least 18 carbon atoms, preferably between 16 and 24 carbon atoms, more preferably between 16 and 20 carbon atoms.

A further object of the disclosure is a PHFA comprising more than 50%, preferably more than 60%, preferably more than 70%, preferably more than 80%, preferably more than 90%, preferably more than 95%, in particular around 100%, of hydroxylated fatty acids monomers comprising at least 16 carbon atoms, preferably at least 18 carbon atoms, preferably between 16 and 24 carbon atoms, more preferably between 16 and 20 carbon atoms.

The term "around" designates in the present invention a range ±1% around the value.

Each PHFA obtained by the method of the invention has a molecular weight of at least 500 g/mol.

Preferably, a PHFA according to the invention has a molecular weight (mass-average molar mass) of at least 1250 g/mol, at least 2500 g/mol, at least 12 500 g/mol, at least 25 000 g/mol, at least 50 000 g/mol, at least 100 000 g/mol, at least 150 000 g/mol.

Preferably, the polydispersity index of a PHFA according to the invention is lower than three, preferably lower than 2.5, more preferably lower than 2. As is well known in the art, the polydispersity index is the ratio of the mass-average molar mass to the number-average molar mass of the polymer.

The thermal properties of the PHFAs of the invention may be analyzed by thermogravimetric analysis (TGA) or by differential scanning calorimetry (DSC).
In an embodiment, the melting point of a PHFA according to the invention is in the range of 50 to 150°C, preferably in the range of 70 to 120°C.

The PHFAs of the invention can be characterized by their advantageous mechanical and/or rheological properties. In particular, the PHFAs of the invention may be characterized by a high tensile modulus, a high elongation to break, and/or a high shear viscosity.

The term "polymerization" designates a chemical reaction wherein the molecules of at least one monomer are linked together to form large molecules whose molecular weight is a sum of the molecular weight of each monomer.
A "homopolymer" is a polymer comprising only one type of monomers. A "heteropolymer" is a polymer comprising at least two different monomers.

### Step d: recovery of the PHFA

The last step of the method of the invention is the recovery of the PHFA from the reaction medium, in particular from the microorganism wherein step c was performed. The action of recovering PHFA is well known by one of ordinary skill in the art. The recovery of the PHFA(s) can be performed by extraction from the microorganism by means of an organic solvent, or the protein material of the microorganism may be decomposed using an aqueous route, leaving PHFAs particles. A useful method for extraction is given in EP 0 145 233. In particular, after the producing cells are collected by centrifugation and freeze-dried, polymers accumulated in the strains can be recovered using solvents, such as chloroform, tetrahydrofuran (THF), dimethylsulfoxide (DMSO), or hexafluoroisopropanol. The recovered PHFA may be subsequently precipitated in a short-chain alcohol, such as methanol or ethanol, or a mixture thereof; the precipitate obtained thereby may be separated by centrifugation, re-dissolved in an organic solvent such as chloroform, and re-precipitated at least once to increase its purity.
An alternative method for recovering the PHFA from the culture medium is to use, at least for the polymerization step of the method of the invention, a microorganism comprising an enzyme that is appropriate for extracting the PHFA from the microorganism, such as a *Pseudomonas putida* KT2440 which is mutant in one or more of the *tol-pal* genes as described in the international patent application WO 2011/086211.

The percentage values in the present invention are weight percentages, except if they are specifically defined as not being weight percentages.

The following examples must be considered as illustrative, and not limitative, of the present invention.

### Examples

### Example 1: Production of PHFA from oils with an engineered E. Coli strain using the β-oxidation pathway

### Hydrolysis of oils with a lipase :

Rapeseed oil; sunflower oil and palm oil are treated separately with 1 % (v/v) of Lipozyme CalB L (Novozymes, Denmark) at 50°C for 5 h, allowing the hydrolysis of glycerol and fatty acids.

The composition of fatty acid is determined after methylation according to the DIN EN14105:2011-07 (Fat and oil derivatives - Fatty Acid Methyl Esters (FAME) - Determination of free and total glycerol and mono-, di-, and triglyceride contents", European Committee for Standardization) using gas chromatography (Agilent 7890 Series GC).

**Table 1 : Analysis of fatty acid composition after hydrolysis of rapeseed oil, sunflower oil and palm oil with Lipozyme calB L :**

| Fatty acid | Rapeseed oil (%) | Sunflower oil (%) | Palm oil (%) |
|---|---|---|---|
| C14:0 | 0 | 0 | 2.0 |
| C16:0 | 3.0 | 4.7 | 40.5 |
| C18:0 | 1.5 | 4.0 | 3.5 |
| C18:1 | 34.5 | 70.3 | 42.0 |
| C18:2 | 20.0 | 19.7 | 10.0 |
| C18:3 | 5.0 | 0 | 0.5 |
| C20:0 | 1.0 | 0.2 | 1.0 |
| C20:1 | 8.0 | 0.2 | 0.3 |
| C22:0 | 0.5 | 0.6 | 0.2 |
| C22:1 | 25.5 | 0 | 0 |
| C24:0 | 0.5 | 0.3 | 0 |
| C24:1 | 0.5 | 0 | 0 |

### Deletion of FadA, FadB and FadI genes in E. coli K-12 MG1655

Gene knockouts of FadA, FadB and FadI are introduced in MG1655 by P1 phage transduction as described by Miller, 1972 (J.H. Miller, Experiments in Molecular Genetics, Cold Spring Harbor Laboratory Press, Cold Spring Harbor, NY (1972)). The strain obtained is called *E. coli* MG1655 ΔFadABI.

### Cloning of phaJ4 and PHA C2 genes from Pseudomonas aeruginosa in E. coli MG1655 ΔFad4BI

Standard molecular genetics techniques are used for the cloning and transformation in strain *E. coli* MG1655 ΔFadABI (Sambrook et al. 1989, Molecular cloning: a laboratory manual (Cold Spring Harbor Lab. Press, Plainview, NY) 2nd Ed.)

PhaJ4 (SEQ ID n°1) and PHA C2 genes (SEQ ID N°3) are amplified from *P*. *aeruginosa* genomic DNA by PCR using primers described in SEQ ID n°7, SEQ ID n°8, SEQ ID n°9 and SEQ ID n°10.

PCR product corresponding to PhaJ4 gene and plasmid pET21a (Novagen, Merck Millipore) are then digested with restriction enzymes NheI and BamHI (New England Biolabs, Evry, France). After digestion, the insert and vector are purified using the QIAquick Gel Extraction kit (Qiagen). Ligation is done using T4 DNA ligase (1 h, room temperature). Transformation of chemio-competent *E. coli* DH5α is performed using recommendations of the provider. After transformation, transformed bacteria are spread on LB Agar medium containing ampicillin (100 µg/mL) in order to select recombinant bacteria transformed with the ligation product. Positive colonies are cultivated one night at 37°C in LB medium containing ampicillin (100 µg/mL). Amplified vector is purified using QIAprep Spin Miniprep Kit (Qiagen) and checked by sequencing. pET21a vector containing PhaJ4 gene is named pET21-PhaJ4.

PCR product corresponding to PHA C2 gene and plasmid pET21-PhaJ4 are digested with restriction enzymes EcoRI and HindIII (New England Biolabs, Evry, France). After digestion, the insert and vector are purified using the QIAquick Gel Extraction kit (Qiagen). Ligation is done using T4 DNA ligase (1 h, room temperature). Transformation of chemio-competent *E*. *coli* DH5α is performed using recommendations of the provider. After transformation, transformed bacteria are spread on LB Agar medium containing ampicillin (100 µg/mL) in order to select recombinant bacteria transformed with the ligation product. Positive colonies are cultivated one night at 37°C in LB medium containing ampicillin (100 µg/mL). Amplified vector is purified using QIAprep Spin Miniprep Kit (Qiagen) and checked by sequencing. pET21a vector containing PhaJ4 gene and PHA C2 gene is named pET21-PhaJ4-PHA C2.

Vector pET21-PhaJ4-PHA C2 is transformed in *E. coli* MG1655 ΔFadABI and strain expressing PhaJ4 and PHA C2 are selected on LB Agar + ampicillin. The recombinant strain is named *E. coli* MG1655 ΔFadABI PhaJ4-PHA C2.

### Culture of strain E. coli MG1655 ΔFadABI PhaJ4 PHA C2 on Hydrolyzed oils

3 bioreactor experiments are performed in a 7.5 L stirred bioreactor (Labfors 5, Infors) using a 5.0 L working volume of M9 medium containing each hydrolyzed oil (composition below). Temperature is maintained at 37 °C. Temperature, pH, and dissolved oxygen are monitored using specific probes. Vessel pH was maintained at 7.20±0.1 by addition of 1 M KOH or 1 M HCl solutions. Agitation is provided by a single impeller. Stirrer speed is regulated in order to maintain the dissolved oxygen content above 40% saturation in order to keep an aerobic environment. Air inflow is maintained at 2.0 L min⁻¹.

Preculture of strain *E. coli* MG1655 ΔFadABI PhaJ4-PHA C2 is performed overnight in 50 mL LB medium containing ampicillin at 37°C and 150 rpm.

### Composition of medium M9 + hydrolyzed oil:

| Hydrolyzed oil | 10 % (v/v) |
|---|---|
| disodium phosphate heptahydrate | 12.8 g/L |
| monopotassium phosphate | 3 g/L |
| ammonium chloride | 1 g/L |
| sodium chloride | 0.5 g/L |
| magnesium sulfate heptahydrate | 2 mM |

Bioreactor experiments are inoculated at an optical density (OD at 600 nm) of 0.05 with a culture of strain *E. coli* MG1655 ΔFadABI PhaJ4-PHA C2. Induction with 1 mM IPTG occurs when the OD 600 of the bioreactor reaches 1.0. The reactor is operated in batch mode. The OD 600 of the culture is monitored periodically. The content of the bioreactor is harvested at 48 h post-induction for PHFA analysis.

### Recovery of PHFA

After fermentation, PHFA produced are recovered using the method developed by Jiang et al., 2006 ("Acetone extraction of mcl-PHA from Pseudomonas putida KT2440", Journal of Microbiological Methods 67 (2006) 212-219). PHFA molecular weight determination is performed on a gel permeation chromatography (Agilent 1260 Infinity GPC/SEC System) using serial columns Shodex K-80M and K-802. Chloroform is used as eluent at a flow rate of 0.5 mL/min. Sample concentration of 1 mg/mL are applied. Polystyrene standards are used for calibration. Analysis of the composition of PHFA is performed by GC/MS based on the method of Kato et al. (Appl. Microbiol. Biotechnol. 1996, 45, 363).

### Results : composition of PHFA Produced after 48 h of culture on hydrolyzed oils :

PHFAs comprising more than 50 % of long chain length β-hydroxylated fatty acids are obtained after 48h of culture of strain *E. coli* MG1655 ΔFadABI PhaJ4-PHA C2 on hydrolyzed oils. For culture of recombinant *E. coli* on rapeseed oil, long chain length β-hydroxylated fatty acids are mainly 3-hydroxyoctadecanoic acid and 3-hydroxydocosanoic acid. For culture on sunflower oil, long chain length β-hydroxylated fatty acids are mainly 3-hydroxyoctadecanoic acid. For culture on palm oil, long chain length β-hydroxylated fatty acids are mainly 3-hydroxyhexadecanoic acid and 3-hydroxyoctadecanoic acid.

### Example 2: Production of PHFA from oils with engineered Pseudomouas oleovoraus

### Hydrolysis of oils with a lipase :

Rapeseed oil, sunflower oil and palm oil are treated separately with 1 % (v/v) of Lipozyme CalB L (Novozymes, Denmark) at 50°C for 5 h, allowing the hydrolysis of glycerol and fatty acids, as described in example 1.

### Deletion of FadA and PHA C1 genes in Pseudomonas oleovorans gPo1.

The method for gene knock out is derived from the method described by Schäfer *et al.,* 1994 (Schäfer A, Tauch A, Jäger W, Kalinowski J, Thierbach G, Pühler A, (1994) Small mobilizable multi-purpose cloning vectors derived from the Escherichia coli plasmids pK18 and pK19: selection of defined deletions in the chromosome of Corynebacterium glutamicum. Gene 145:69-73).

FadA (SEQ ID n°11) and PHA C1 (SEQ ID N°12) genes are amplified from *P. aeruginosa* genomic DNA by PCR using primers described in SEQ ID n°13, SEQ ID n°14, SEQ ID n°15 and SEQ ID n°16.

PCR product corresponding to fadA and PHA C1 genes are purified using QIAquick Gel Extraction kit (Qiagen) before being digested with restriction enzymes XbaI and HindIII (New England Biolabs, Evry, France). In parallel, plasmid pK18mobsacB is digested with the same restriction enzymes. FadA and PHA C1 inserts are ligated separately into digested pK18mobsacB plasmid. The new plasmids containing FadA gene and PHA C1 gene are called pSFadA and pSPHAC1 respectively. pSFasA and pSPHAC1 are digested with SacII and EagI respectively; The larger fragments are self-ligated to form new plasmids called pSΔFadA and pSΔPHAC1. Plasmids are transformed into *E. coli* DH5a strains by electroporation. Transformed strains are selected on LB medium containing kanamycin.

Conjugation with *Pseudomonas aeruginosa* gPo1 is done as described by Stanisich and Holloway (Stanisich V, Holloway BW, "Conjugation in Pseudomonas aeruginosa", Genetics. 1969 Feb;61(2):327-39.).

The strain of *Pseudomonas aeruginosa* gPo1 deleted of genes FadA and PHA C1 is called *P. aeruginosa* ΔFadA-APHAC1.

### Culture of strain P. aeruginosa ΔFadA-ΔPHAC1 on Hydrolyzed oils

3 bioreactor experiments are performed in a 7.5 L stirred bioreactor (Labfors 5, Infors) using a 5.0 L working volume of dedicated medium containing each hydrolyzed oils (composition below). Temperature is maintained at 30 °C. Temperature, pH, and dissolved oxygen are monitored using specific probes. Vessel pH was maintained at 6.5±0.2 by addition of 1 M KOH or 1 M HCl solutions. Agitation is provided by a single impeller. Stirrer speed is regulated in order to maintain the dissolved oxygen content above 30% saturation in order to keep an aerobic environment.

### Composition of medium for P aeruginosa + hydrolyzed oil:

| Hydrolyzed oil | 10 % (v/v) |
|---|---|
| Yeast extract | 5 g/L |
| Tryptone | 10 g/L |
| Ammonium sulfate | 4 g/L |
| Sodium chloride | 10 g/L |
| Disodium phosphate | 2 g/L |
| Potassium phosphate | 1.5 |
| Magnesium sulfate heptahydrate | 0.5 g/L |

Bioreactor experiments are inoculated at an optical density (OD 600 nm) of 0.05 with a culture of strain P. aeruginosa ΔFadA-ΔPHAC1. The reactor is operated in fed-batch mode by addition of hydrolyzed oil when glycerol consumption is below 0.05 g/L. The OD 600 of the culture is monitored periodically. The content of the bioreactor is harvested after 96 h of cultivation for PHFA analysis.

### Recovery of PHFA and analysis of their composition

After fermentation, PHFA produced are recovered using the method developed by Jiang et al., 2006 ("Acetone extraction of mcl-PHA from Pseudomonas putida KT2440", Journal of Microbiological Methods 67 (2006) 212-219). PHFA molecular weight determination is performed on a gel permeation chromatography (Agilent 1260 Infinity GPC/SEC System) using serial columns Shodex K-80M and K-802. Chloroform is used as eluent at a flow rate of 0,5 mL/min. Sample concentration of 1 mg/mL are applied. Polystyrene standards are used for calibration. Analysis of the composition of PHFA is performed by GC/MS based on the method of Kato *et al.* (1996).

### Results : composition of PHFA produced after 96 h of culture on hydrolyzed oils :

PHFAs comprising more than 50 % of long chain length β-hydroxylated fatty acids are obtained after 96h of culture of strain *P. aeruginosa* ΔFadA-ΔPHAC1 on hydrolyzed oils. For culture on rapeseed oil, long chain length β-hydroxylated fatty acids are mainly 3-hydroxyoctadecanoic acid and 3-hydroxydocosanoic acid. For culture on sunflower oil, long chain length β-hydroxylated fatty acids are mainly 3-hydroxyoctadecanoic acid. For culture on palm oil, long chain length β-hydroxylated fatty acids are mainly 3-hydroxyhexadecanoic acid and 3-hydroxyoctadecanoic acid.

### Example 3 : Production of ω-hydroxy fatty acids with E. Coli and synthesis of PHFA

### Hydrolysis of oils with a lipase :

Rapeseed oil, sunflower oil and palm oil are treated separately with 1 % (v/v) of Lipozyme CalB L (Novozymes, Denmark) at 50°C for 5 h, allowing the hydrolysis of glycerol and fatty acids, as described in example 1.

### Cloning of CYP102A1 gene from Bacillus megaterium BM-3 in E. coli BL21 (DE3)

Standard molecular genetics techniques are used for the cloning and transformation in strain *E. coli* BL21 (DE3) (Sambrook et al. 1989). Cloning of CYP102A1 is done as described by Pflug *et al.,* 2007 (Simon Pflug, Sven M. Richter, Vlada B. Urlacher, Development of a fed-batch process for the production of the cytochrome P450 monooxygenase CYP102A1 from Bacillus megaterium in E. coli, Journal of Biotechnology, 129, 3, 481-488).

Gene corresponding to cytochrome P450 from *Bacillus megaterium* BM3 (CYP102A1, SEQ ID n°5) is amplified from *B. megaterium* genomic DNA using primers SEQ ID n°17 and SEQ ID n°18.

PCR product corresponding to CYP102A1 gene and plasmid pET28a (Novagen, Merck Millipore) are then digested with restriction enzymes NcoI and BamHI (New England Biolabs, Evry, France). After digestion, the insert and vector are purified using the QIAquick Gel Extraction kit (Qiagen). Ligation is done using T4 DNA ligase (1 h, room temperature). Transformation of chemio-competent *E. coli* DH5α is performed using recommendations of the provider. After transformation, transformed bacteria are spread on LB Agar medium containing kanamycin (40 µg/mL) in order to select recombinant bacteria transformed with the ligation product. Positive colonies are cultivated one night at 37°C in LB medium containing kanamycin (40 µg/mL). Amplified vector is purified using QIAprep Spin Miniprep Kit (Qiagen) and checked by sequencing. pET28a vector containing CYP102A1 gene is named pET28-CYP102A1. Vector pET28-CYP102Alis transformed in *E. coli* BL21 (DE3) by heat shock. The recombinant strain is named *E. coli* BL21 (DE3)-CYP102A1.

### Deletion of FadD gene in E. coli BL21 (DE3)-CYP102A1.

Gene knockout of FadD is introduced in *E. coli* BL21 (DE3)-CYP102A1 by P1 phage transduction as described by Miller, 1972 (J.H. Miller, Experiments in Molecular Genetics, Cold Spring Harbor Laboratory Press, Cold Spring Harbor, NY (1972)). The strain obtained is called *E. coli* BL21 (DE3)-CYP102A1 ΔFadD.

### Culture of strain E. coli BL21 (DE3)-CYP102A1 ΔFadD on Hydrolyzed oils

Precultures of strain *E. coli* BL21 (DE3)-CYP102A1 ΔFadD are performed overnight in 50 mL LB medium containing ampicillin at 37°C and 150 rpm.

3 bioreactor experiments are performed in a 7.5 L stirred bioreactor (Labfors 5, Infors) using a 5.0 L working volume of TB medium containing each hydrolyzed oil (composition below). Temperature is maintained at 37 °C during growth phase and 30°C after induction with IPTG (0.25 mM). Temperature, pH, and dissolved oxygen are monitored using specific probes. Vessel pH was maintained at 7.20±0.1 by addition of KOH or H₃PO₄ solutions. Agitation is provided by a Rushton impeller. Stirrer speed and air inflow are regulated in order to maintain the dissolved oxygen content below 2 % in order to keep an anaerobic environment and avoid the oxidation of hydroxyl fatty acid formed into keto-fatty acids.

### Composition of medium TB + hydrolyzed oil:

| Hydrolyzed oil | 5 % (v/v) |
|---|---|
| Tryptone | 12 g/L |
| Yeast extract | 24 g/L |
| Potassium phosphate monobasic | 2.3 g/L |
| Dipotassium phosphate | 12.5 g/L |

Bioreactor experiments are inoculated at an optical density (OD at 600 nm) of 0.05 with a preculture of strain *E. coli* BL21 (DE3)-CYP102A1 ΔFadD. Induction with 0.25 mM IPTG occurs when the OD 600 of the bioreactor reaches 1.0. The reactor is operated in fed-batch mode by addition of hydrolyzed oil when glycerol consumption is below 0.1 g/L. The OD 600 of the culture is monitored periodically. The content of the bioreactor is harvested at 48 h post-induction for recovery of hydroxyl fatty acids.

### Recovery of long chain length β-hydroxyfatty acids produced by strain E. coli BL21 (DE3)-CYP102A1 ΔFadD on different hydrolyzed oils.

After fermentation, bacterial cells are recovered by centrifugation. Supernatant is discarded. Cells are washed twice with 0.9 % NaCl solution in order to remove all remaining trace of hydrolyzed oils. Cells are resuspended in potassium phosphate buffer containing 2 mM Dithiothreitol (DTT) are disrupted by using a French press. Cell debris are discarded by centrifugation.

### Synthesis of PHFA from long chain length β-hydroxyfatty acids with a lipase

Novozym435 (immobilized CalB from Novozymes) is used for polymerization of long chain length β-hydroxyfatty acids in diphenyl ether at 60°C under vacuum for at least 48 h. After polymerization, diphenyl ether is evaporated and PHFA are recovered in chloroform.

### Analysts of PHFA

PHFA molecular weight determination is performed on a gel permeation chromatography (Agilent 1260 Infinity GPC/SEC System) using serial columns Shodex K-80M and K-802. Chloroform is used as eluent at a flow rate of 0.5 mL/min. Sample concentration of 1 mg/mL are applied. Polystyrene standards are used for calibration. Hydrolysis of PHFA and transmethylation of monomers are performed as described by Kato et al. (Appl. Microbiol. Biotechnol. 1996, 45, 363). The analysis of the composition of PHFAs by GC/MS indicates that PHFA produced with hydrolyzed oils contains more than 50 % of long chain length β-hydroxylated fatty acids (ω-hydroxyhexadecanoic acid, ω-hydroxyoctadecanoic acid and ω-hydroxydocosanoic acid depending on the hydrolyzed oil used as substrate).

## Claims

1. A method for the preparation of at least one long-chain PHFA, comprising the steps of:
a) Obtaining of long chain length fatty acids comprising at least 16 carbon atoms from oil,
b) Oxidation of long chain length fatty acids to form hydroxy(fatty acids) or derivatives thereof such as CoA thioesters thereof,
c) Polymerization of the hydroxy(fatty acids) or derivatives thereof obtained in step b to form at least one PHFA, and
d) Recovery of the at least one PHFA obtained in step c,
wherein step b) is a ω oxidation, performed in a recombinant microorganism comprising at least one appropriate cytochrome P450 enzyme.

2. The method according to claim 1, wherein oil is a vegetable oil, or a mixture of such oils, preferably chosen in the group consisting of: rapeseed oil, sunflower oil, peanut oil, corn oil, olive oil, cotton seed oil, sesame oil, palm oil, soybean oil, mustard oil and a mixture thereof.

3. The method according to claim 1 or 2, wherein the oil comprises triglycerides with long fatty chains comprising from 16 to 24 carbon atoms.

4. The method according the claims 1 to 3, wherein step a) comprises a transformation of at least some of the triglycerides comprised in the oil into long chain length fatty acids and glycerol.

5. The method according to any of claims 1 to, wherein step b) involves a microorganism selected among *Cupriavidus necator, Escherichia coli, Pseudomonas aeruginosa, Pseudomonas putida, Pseudomonas entomophila, Pseudomonas oleovorans, Yarrowia lipolytica, Aeromonas hydrophila, Candida tropicalis, Candida antarctica, Burkholderia xenovorans, Burkholderia mallei, Burkholderia pseudomallei, Saccharomyces cerevisiae, Bacillus subtilis* and *Bacillus megaterium.*

6. The method according to claim 5, wherein the genes coding for the enzymes implied in the degradation of CoA thioesters of β hydroxylated fatty acids are silenced or deleted in the microorganism.

7. The method according to any of claims 5 to 6, wherein the genes coding for the enzymes implied in the formation of CoA thioesters of β hydroxylated fatty acids are added or enhanced in the microorganism.

8. The method according to any of claims 1 to 7, wherein step c) is performed in a microorganism, preferably with a PHA synthase.

9. The method according to any of claims 1 to 8, wherein step c) is performed in extracellular conditions, preferably in presence of a lipase.

10. The method according to any of claims 1 to 9, wherein step d) is performed with an organic solvent chosen among chloroform, tetrahydrofuran, dimethylsulfoxide, or hexafluoroisopropanol.

11. The method according to any of claims 1 to 10, wherein the recombinant microorganism comprises at least one cytochrome P450 enzyme selected from CYP52A from *Candida tropicalis,* CYP94A1 from *Vicia sativa,* CYP 94A5 from *Nicotiana tabacum,* CYP78A1 from *Zea mays,* CYP 86A1 and CYP86A8 from *Arabidopsis thaliana,* CYP 92B1 from *Petunia hybrida,* CYP102A1 (BM-3) mutant F87 from *Bacillus megaterium,* CYP 4 family from mammal and insect, CYP81B1 from *Helianthus tuberosus* (ω-1 to ω-5 hydroxylation), CYP790C1 from *Helianthus tuberosus* (ω-1 and ω-2 hydroxylation), CYP152B1 *from Sphingomonas paucimobilis* (α-hydroxylation), CYP2E1 and 4A1 from human liver (ω-1 hydroxylation); P450BSβ from *Bacillus subtilis* (α and β-hydroxylation), and CYP102A1 (BM-3) from *Bacillus megaterium* (ω-1, ω-2 and ω-3 hydroxylation.

## Patentansprüche

1. Ein Verfahren zur Herstellung mindestens einer langkettigen PHFA, umfassend die folgenden Schritte:
a) Gewinnen von langkettigen Fettsäuren, die mindestens 16 Kohlenstoffatome umfassen, aus Öl,
b) Oxidation der langkettigen Fettsäuren, um Hydroxy(Fettsäuren) oder Derivate davon wie CoA-Thioester davon zu bilden,
c) Polymerisierung der in Schritt b erhaltenen Hydroxy(Fettsäuren) oder der Derivate davon, um mindestens eine PHFA zu bilden, und
d) Rückgewinnung der in Schritt c erhaltenen mindestens einen PHFA,
wobei Schritt b) eine ω-Oxidation ist, durchgeführt in einem rekombinanten Mikroorganismus, der mindestens ein geeignetes Cytochrom P450-Enzym umfasst.

2. Das Verfahren gemäß Anspruch 1, wobei das Öl ein Gemüseöl ist, oder ein Gemisch solcher Öle, vorzugsweise ausgewählt aus der Gruppe bestehend aus: Rapsöl, Sonnenblumenöl, Erdnussöl, Maiskeimöl, Olivenöl, Baumwollsamenöl, Sesamöl, Palmöl, Sojabohnenöl, Senföl und einem Gemisch davon.

3. Das Verfahren gemäß Anspruch 1 oder 2, wobei das Öl Triglyceride umfasst, die lange Fettketten von 16 bis 24 Kohlenstoffatomen umfassen.

4. Das Verfahren gemäß einem der Ansprüche 1 bis 3, wobei Schritt a) eine Transformation zumindest einiger der in dem Öl umfassten Triglyceride in Fettsäuren mit langer Kettenlänge und Glycerin umfasst.

5. Das Verfahren gemäß einem der Ansprüche 1 bis, wobei Schritt b) einen Mikroorganismus nutzt, der aus *Cupriavidus necator, Escherichia coli, Pseudomonas aeruginosa, Pseudomonas putida, Pseudomonas entomophila, Pseudomonas oleovorans, Yarrowia lipolytica, Aeromonas hydrophila, Candida tropicalis, Candida antarctica, Burkholderia xenovorans, Burkholderia mallei, Burkholderia pseudomallei, Saccharomyces cerevisiae, Bacillus subtilis* und *Bacillus megaterium* ausgewählt ist.

6. Das Verfahren nach Anspruch 5, wobei die Gene, die für diejenigen Enzyme kodieren, die am Abbau von CoA-Thioestern β-hydroxylierter Fettsäuren beteiligt sind, in dem Mikroorganismus ausgeschaltet sind oder entfernt wurden.

7. Das Verfahren gemäß einem der Ansprüche 5 bis 6, wobei die Gene, die für diejenigen Enzyme kodieren, die am Aufbau von CoA-Thioestern β-hydroxylierter Fettsäuren beteiligt sind, in dem Mikroorganismus hinzugefügt oder verstärkt wurden.

8. Das Verfahren gemäß einem der Ansprüche 1 bis 7, wobei Schritt c) in einem Mikroorganismus durchgeführt wird, vorzugsweise mit einer PHA-Synthase.

9. Das Verfahren gemäß einem der Ansprüche 1 bis 8, wobei Schritt c) unter extrazellulären Bedingungen durchgeführt wird, vorzugsweise in Gegenwart einer Lipase.

10. Das Verfahren gemäß einem der Ansprüche 1 bis 9, wobei Schritt d) in einem organischen Lösungsmittel durchgeführt wird, das aus Chloroform, Tetrahydrofuran, Dimethylsulfoxid oder Hexafluorisopropanol ausgewählt ist.

11. Das Verfahren gemäß einem der Ansprüche 1 bis 10, wobei der rekombinante Mikroorganismus mindestens ein Cytochrom P450-Enzym ausgewählt aus CYP52A aus *Candida tropicalis,* CYP94A1 aus *Vicia sativa,* CYP 94A5 aus *Nicotiana tabacum,* CYP78A1 aus *Zea mays,* CYP 86A1 und CYP86A8 aus *Arabidopsis thaliana,* CYP 92B1 aus *Petunia hybrida,* CYP102A1 (BM-3)-Mutante F87 aus *Bacillus megaterium,* CYP 4-Familie der Säuger und Insekten, CYP81B1 aus *Helianthus tuberosus* (ω-1- bis ω-5-Hydroxylierung), CYP790C1 aus *Helianthus tuberosus* (ω-1- und ω-2-Hydroxylierung), CYP152B1 aus *Sphingomonas paucimobilis* (α-Hydroxylierung), CYP2E1 und 4A1 aus der menschlichen Leber (ω-1-Hydroxylierung); P450BSß aus *Bacillus subtilis* (α- und β-Hydroxylierung), und CYP102A1 (BM-3) aus *Bacillus megaterium* (ω-1-, ω-2- und ω-3-Hydroxylierung) umfasst.

## Revendications

1. Méthode de préparation d'au moins un PHFA à longue chaine, comprenant les étapes consistant à :
a) Obtenir des acides gras à longue chaine comprenant au moins 16 atomes de carbone à partir d'huile ;
b) Oxyder les acides gras à longue chaine pour former des hydroxy-acides gras ou des dérivés de ceux-ci tels que des thioesters CoA de ceux-ci ;
c) Polymériser les hydroxy-acides gras or dérivés de ceux-ci obtenus à l'étape b pour former au moins un PHFA; et
d) Récupérer le au moins un PHFA de l'étape c,
dans laquelle l'étape b) est une ω oxydation, réalisée par un microorganisme recombinant comprenant au moins une enzyme cytochrome P450 appropriée.

2. Méthode selon la revendication 1, dans laquelle l'huile est une huile végétale, ou un mélange de telles huiles, préférentiellement choisie dans le groupe constituer de huile de colza, huile de tournesol, huile de cacahuètes, huile de maïs, huile d'olive, huile de graine de coton, huile de sésame, huile de palme, huile de soja, huile de moutarde, ou un mélange de celles-ci.

3. Méthode selon la revendication 1 ou 2, dans laquelle l'huile comprend des triglycérides à chaines grasses longues comprenant de 16 à 24 atomes de carbone.

4. Méthode selon l'une des revendications 1 à 3, dans laquelle l'étape a) comprend une transformation d'au moins certains triglycérides compris dans l'huile en des acides gras à longue chaine et du glycérol.

5. Méthode selon la revendication 1, dans laquelle l'étape b) implique un microorganisme sélectionné parmi *Cupriavidus necator, Escherichia coli, Pseudomonas aeruginosa, Pseudomonas putida, Pseudomonas entomophila, Pseudomonas oleovorans, Yarrowia lipolytica, Aeromonas hydrophila, Candida tropicalis, Candida antarctica, Burkholderia xenovorans, Burkholderia mallei, Burkholderia pseudomallei, Saccharomyces cerevisiae, Bacillus subtilis* et *Bacillus megaterium.*

6. méthode selon la revendication 5, dans laquelle les gènes codant pour les enzymes impliqués dans la dégradation des thioesters CoA des acides gras β hydroxylés sont silencieux ou délétés dans le microorganisme.

7. Méthode selon la revendication 5 à 6, dans laquelle les gènes codant pour les enzymes impliqués dans la formation des thioesters CoA des acides gras β hydroxylés sont ajoutés ou augmentés dans le microorganisme.

8. Méthode selon l'une quelconque des revendications 1 à 7, dans laquelle l'étape c) est réalisée dans un microorganisme, préférentiellement avec une PHA synthase.

9. Méthode selon l'une quelconque des revendications 1 à 7, dans laquelle l'étape c) est réalisée dans des conditions extracellulaires, préférentiellement en présence d'une lipase.

10. Méthode selon l'une quelconque des revendications 1 à 9, dans laquelle l'étape d) est réalisée avec un solvant organique choisi parmi le chloroforme, le tetrahydrofurane, le diméthylsulfoxyde ou l'hexafluoroisopropanole.

11. Méthode selon l'une quelconque des revendications 1 à 10, dans laquelle le microorganisme recombinant comprend au moins une enzyme cytochrome P450 sélectionnée parmi CYP52A de *Candida tropicalis,* CYP94A1 de *Vicia sativa,* CYP 94A5 de *Nicotiana tabacum,* CYP78A1 de *Zea mays,* CYP 86A1 et CYP86A8 de *Arabidopsis thaliana,* CYP 92B1 de *Petunia hybrida,* CYP102A1 (BM-3) mutant F87 de *Bacillus megaterium,* famille CYP 4 de mammifère ou d'insecte, CYP81B1 de *Helianthus tuberosus* (ω-1 à ω-5 hydroxylation), CYP790C1 de *Helianthus tuberosus* (ω-1 et ω-2 hydroxylation), CYP152B1 *de Sphingomonas paucimobilis* (α-hydroxylation), CYP2E1 et 4A1 de foie humain (ω-1 hydroxylation); P450BSβ de *Bacillus subtilis* (α et β-hydroxylation), et CYP102A1 (BM-3) de *Bacillus megaterium* (ω-1, ω-2 et ω-3 hydroxylation).


### SEQUENCE LISTING

<110> CARBIOS
<120> Preparation of long-chain length poly(hydroxyfatty acids)
<130> B1524PC00
<160> 18
<170> PatentIn version 3.5
<210> 1
   <211> 456
   <212> DNA
   <213> Pseudomonas aeruginosa
<400> 1
   atgccattcg tacccgtagc agcgctcaag gactatgtcg gcaaggatct cggccattcc        60
   gaatggctca ccatcgacca ggagcgggtc gaccagttcg ccgaatgcac cggcgaccac       120
   cagttcatcc acgtcgatcc ggagaaggcg gcgaagacgc ccttcggcgg caccatcgcc       180
   cacggtttcc tgtccctgtc gctgatcccg aagctgatgg aaggcctgct ggtgcttccc       240
   gaaggactga agatggcggt caactacggc ctcgatacgg tgcgcttcat ccagccggtg       300
   cgggtcggct cgcgggtccg cctgggcctg accctgctcg acgtcaacga gaagaatccc       360
   ggccagtggc tgatcaaggc gcgcgccacc ctggagatcg aaggccagga gaaacccgcc       420
   tacatcgccg agaccctcag cctctgcttc gtctga                                 456
<210> 2
   <211> 151
   <212> PRT
   <213> Pseudomonas aeruginosa
<400> 2
   Met Pro Phe Val Pro Val Ala Ala Leu Lys Asp Tyr Val Gly Lys Asp
   1               5                   10                  15
   Leu Gly His Ser Glu Trp Leu Thr Ile Asp Gln Glu Arg Val Asp Gln
               20                  25                  30
   Phe Ala Glu Cys Thr Gly Asp His Gln Phe Ile His Val Asp Pro Glu
           35                  40                  45
   Lys Ala Ala Lys Thr Pro Phe Gly Gly Thr Ile Ala His Gly Phe Leu
       50                  55                  60
   Ser Leu Ser Leu Ile Pro Lys Leu Met Glu Gly Leu Leu Val Leu Pro
   65                  70                  75                  80
   Glu Gly Leu Lys Met Ala Val Asn Tyr Gly Leu Asp Thr Val Arg Phe
                   85                  90                  95
   Ile Gln Pro Val Arg Val Gly Ser Arg Val Arg Leu Gly Leu Thr Leu
               100                 105                 110
   Leu Asp Val Asn Glu Lys Asn Pro Gly Gln Trp Leu Ile Lys Ala Arg
           115                 120                 125
   Ala Thr Leu Glu Ile Glu Gly Gln Glu Lys Pro Ala Tyr Ile Ala Glu
       130                 135                 140
   Thr Leu Ser Leu Cys Phe Val
   145                 150
<210> 3
   <211> 1683
   <212> DNA
   <213> Pseudomonas aeruginosa
<400> 3
   atgcgagaaa agcaggaatc gggtagcgtg ccggtgcccg ccgagttcat gagtgcacag      60
   agcgccatcg tcggcctgcg cggcaaggac ctgctgacga cggtccgcag cctggctgtc     120
   cacggcctgc gccagccgct gcacagtgcg cggcacctgg tcgccttcgg aggccagttg     180
   ggcaaggtgc tgctgggcga caccctgcac cagccgaacc cacaggacgc ccgcttccag     240
   gatccatcct ggcgcctcaa tcccttctac cggcgcaccc tgcaggccta cctggcgtgg     300
   cagaaacaac tgctcgcctg gatcgacgaa agcaacctgg actgcgacga tcgcgcccgc     360
   gcccgcttcc tcgtcgcctt gctctccgac gccgtggcac ccagcaacag cctgatcaat     420
   ccactggcgt taaaggaact gttcaatacc ggcgggatca gcctgctcaa tggcgtccgc     480
   cacctgctcg aagacctggt gcacaacggc ggcatgccca gccaggtgaa caagaccgcc     540
   ttcgagatcg gtcgcaacct cgccaccacg caaggcgcgg tggtgttccg caacgaggtg     600
   ctggagctga tccagtacaa gccgctgggc gagcgccagt acgccaagcc cctgctgatc     660
   gtgccgccgc agatcaacaa gtactacatc ttcgacctgt cgccggaaaa gagcttcgtc     720
   cagtacgccc tgaagaacaa cctgcaggtc ttcgtcatca gttggcgcaa ccccgacgcc     780
   cagcaccgcg aatggggcct gagcacctat gtcgaggccc tcgaccaggc catcgaggtc     840
   agccgcgaga tcaccggcag ccgcagcgtg aacctggccg gcgcctgcgc cggcgggctc     900
   accgtagccg ccttgctcgg ccacctgcag gtgcgccggc aactgcgcaa ggtcagtagc     960
   gtcacctacc tggtcagcct gctcgacagc cagatggaaa gcccggcgat gctcttcgcc    1020
   gacgagcaga ccctggagag cagcaagcgc cgctcctacc agcatggcgt gctggacggg    1080
   cgcgacatgg ccaaggtgtt cgcctggatg cgccccaacg acctgatctg gaactactgg    1140
   gtcaacaact acctgctcgg caggcagccg ccggcgttcg acatcctcta ctggaacaac    1200
   gacaacacgc ggctgcccgc ggcgttccac ggcgaactgc tcgacctgtt caagcacaac    1260
   ccgctgaccc gcccgggcgc gctggaggtc agcgggaccg cggtggacct gggcaaggtg    1320
   gcgatcgaca gcttccacgt cgccggcatc accgaccaca tcacgccctg ggacgcggtg    1380
   tatcgctcgg ccctcctgct gggcggccag cgccgcttca tcctgtccaa cagcgggcac    1440
   atccagagca tcctcaaccc tcccggaaac cccaaggcct gctacttcga gaacgacaag    1500
   ctgagcagcg atccacgcgc ctggtactac gacgccaagc gcgaagaggg cagctggtgg    1560
   ccggtctggc tgggctggct gcaggagcgc tcgggcgagc tgggcaaccc tgacttcaac    1620
   cttggcagcg ccgcgcatcc gcccctcgaa gcggccccgg gcacctacgt gcatatacgc    1680
   tga                                                                  1683
<210> 4
   <211> 560
   <212> PRT
   <213> Pseudomonas aeruginosa
<400> 4
   Met Arg Glu Lys Gln Glu Ser Gly Ser Val Pro Val Pro Ala Glu Phe
   1               5                   10                  15
   Met Ser Ala Gln Ser Ala Ile Val Gly Leu Arg Gly Lys Asp Leu Leu
               20                  25                  30
   Thr Thr Val Arg Ser Leu Ala Val His Gly Leu Arg Gln Pro Leu His
           35                  40                  45
   Ser Ala Arg His Leu Val Ala Phe Gly Gly Gln Leu Gly Lys Val Leu
       50                  55                  60
   Leu Gly Asp Thr Leu His Gln Pro Asn Pro Gln Asp Ala Arg Phe Gln
   65                  70                  75                  80
   Asp Pro Ser Trp Arg Leu Asn Pro Phe Tyr Arg Arg Thr Leu Gln Ala
                   85                  90                  95
   Tyr Leu Ala Trp Gln Lys Gln Leu Leu Ala Trp Ile Asp Glu Ser Asn
               100                 105                 110
   Leu Asp Cys Asp Asp Arg Ala Arg Ala Arg Phe Leu Val Ala Leu Leu
           115                 120                 125
   Ser Asp Ala Val Ala Pro Ser Asn Ser Leu Ile Asn Pro Leu Ala Leu
       130                 135                 140
   Lys Glu Leu Phe Asn Thr Gly Gly Ile Ser Leu Leu Asn Gly Val Arg
   145                 150                 155                 160
   His Leu Leu Glu Asp Leu Val His Asn Gly Gly Met Pro Ser Gln Val
                   165                 170                 175
   Asn Lys Thr Ala Phe Glu Ile Gly Arg Asn Leu Ala Thr Thr Gln Gly
               180                 185                 190
   Ala Val Val Phe Arg Asn Glu Val Leu Glu Leu Ile Gln Tyr Lys Pro
           195                 200                 205
   Leu Gly Glu Arg Gln Tyr Ala Lys Pro Leu Leu Ile Val Pro Pro Gln
       210                 215                 220
   Ile Asn Lys Tyr Tyr Ile Phe Asp Leu Ser Pro Glu Lys Ser Phe Val
   225                 230                 235                 240
   Gln Tyr Ala Leu Lys Asn Asn Leu Gln Val Phe Val Ile Ser Trp Arg
                   245                 250                 255
   Asn Pro Asp Ala Gln His Arg Glu Trp Gly Leu Ser Thr Tyr Val Glu
               260                 265                 270
   Ala Leu Asp Gln Ala Ile Glu Val Ser Arg Glu Ile Thr Gly Ser Arg
           275                 280                 285
   Ser Val Asn Leu Ala Gly Ala Cys Ala Gly Gly Leu Thr Val Ala Ala
       290                 295                 300
   Leu Leu Gly His Leu Gln Val Arg Arg Gln Leu Arg Lys Val Ser Ser
   305                 310                 315                 320
   Val Thr Tyr Leu Val Ser Leu Leu Asp Ser Gln Met Glu Ser Pro Ala
                   325                 330                 335
   Met Leu Phe Ala Asp Glu Gln Thr Leu Glu Ser Ser Lys Arg Arg Ser
               340                 345                 350
   Tyr Gln His Gly Val Leu Asp Gly Arg Asp Met Ala Lys Val Phe Ala
           355                 360                 365
   Trp Met Arg Pro Asn Asp Leu Ile Trp Asn Tyr Trp Val Asn Asn Tyr
       370                 375                 380
   Leu Leu Gly Arg Gln Pro Pro Ala Phe Asp Ile Leu Tyr Trp Asn Asn
   385                 390                 395                 400
   Asp Asn Thr Arg Leu Pro Ala Ala Phe His Gly Glu Leu Leu Asp Leu
   405 410 415
   Phe Lys His Asn Pro Leu Thr Arg Pro Gly Ala Leu Glu Val Ser Gly
   420 425 430
   Thr Ala Val Asp Leu Gly Lys Val Ala Ile Asp Ser Phe His Val Ala
   435 440 445
   Gly Ile Thr Asp His Ile Thr Pro Trp Asp Ala Val Tyr Arg Ser Ala
   450 455 460
   Leu Leu Leu Gly Gly Gln Arg Arg Phe Ile Leu Ser Asn Ser Gly His
   465 470 475 480
   Ile Gln Ser Ile Leu Asn Pro Pro Gly Asn Pro Lys Ala Cys Tyr Phe
   485 490 495
   Glu Asn Asp Lys Leu Ser Ser Asp Pro Arg Ala Trp Tyr Tyr Asp Ala
   500 505 510
   Lys Arg Glu Glu Gly Ser Trp Trp Pro Val Trp Leu Gly Trp Leu Gln
   515 520 525
   Glu Arg Ser Gly Glu Leu Gly Asn Pro Asp Phe Asn Leu Gly Ser Ala
   530 535 540
   Ala His Pro Pro Leu Glu Ala Ala Pro Gly Thr Tyr Val His Ile Arg
   545 550 555 560
<210> 5
   <211> 3150
   <212> DNA
   <213> Bacillus megaterium
<400> 5
   atgacaatta aagaaatgcc tcagccaaaa acgtttggag agcttaaaaa tttaccgtta      60
   ttaaacacag ataaaccggt tcaagctttg atgaaaattg cggatgaatt aggagaaatc     120
   tttaaattcg aggcgcctgg tcgtgtaacg cgctacttat caagtcagcg tctaattaaa     180
   gaagcatgcg atgaatcacg ctttgataaa aacttaagtc aagcgcttaa atttgtacgt     240
   gattttgcag gagacgggtt atttacaagc tggacgcatg aaaaaaattg gaaaaaagcg     300
   cataatatct tacttccaag cttcagtcag caggcaatga aaggctatca tgcgatgatg     360
   gtcgatatcg ccgtgcagct tgttcaaaag tgggagcgtc taaatgcaga tgagcatatt     420
   gaagtaccgg aagacatgac acgtttaacg cttgatacaa ttggtctttg cggctttaac     480
   tatcgcttta acagctttta ccgagatcag cctcatccat ttattacaag tatggtccgt      540
   gcactggatg aagcaatgaa caagctgcag cgagcaaatc cagacgaccc agcttatgat      600
   gaaaacaagc gccagtttca agaagatatc aaggtgatga acgacctagt agataaaatt      660
   attgcagatc gcaaagcaag cggtgaacaa agcgatgatt tattaacgca tatgctaaac      720
   ggaaaagatc cagaaacggg tgagccgctt gatgacgaga acattcgcta tcaaattatt      780
   acattcttaa ttgcgggaca cgaaacaaca agtggtcttt tatcatttgc gctgtatttc      840
   ttagtgaaaa atccacatgt attacaaaaa gcagcagaag aagcagcacg agttctagta      900
   gatcctgttc caagctacaa acaagtcaaa cagcttaaat atgtcggcat ggtcttaaac      960
   gaagcgctgc gcttatggcc aactgctcct gcgttttccc tatatgcaaa agaagatacg     1020
   gtgcttggag gagaatatcc tttagaaaaa ggcgacgaac taatggttct gattcctcag     1080
   cttcaccgtg ataaaacaat ttggggagac gatgtggaag agttccgtcc agagcgtttt     1140
   gaaaatccaa gtgcgattcc gcagcatgcg tttaaaccgt ttggaaacgg tcagcgtgcg     1200
   tgtatcggtc agcagttcgc tcttcatgaa gcaacgctgg tacttggtat gatgctaaaa     1260
   cactttgact ttgaagatca tacaaactac gagctggata ttaaagaaac tttaacgtta     1320
   aaacctgaag gctttgtggt aaaagcaaaa tcgaaaaaaa ttccgcttgg cggtattcct     1380
   tcacctagca ctgaacagtc tgctaaaaaa gtacgcaaaa aggcagaaaa cgctcataat     1440
   acgccgctgc ttgtgctata cggttcaaat atgggaacag ctgaaggaac ggcgcgtgat     1500
   ttagcagata ttgcaatgag caaaggattt gcaccgcagg tcgcaacgct tgattcacac     1560
   gccggaaatc ttccgcgcga aggagctgta ttaattgtaa cggcgtctta taacggtcat     1620
   ccgcctgata acgcaaagca atttgtcgac tggttagacc aagcgtctgc tgatgaagta     1680
   aaaggcgttc gctactccgt atttggatgc ggcgataaaa actgggctac tacgtatcaa     1740
   aaagtgcctg cttttatcga tgaaacgctt gccgctaaag gggcagaaaa catcgctgac     1800
   cgcggtgaag cagatgcaag cgacgacttt gaaggcacat atgaagaatg gcgtgaacat     1860
   atgtggagtg acgtagcagc ctactttaac ctcgacattg aaaacagtga agataataaa     1920
   tctactcttt cacttcaatt tgtcgacagc gccgcggata tgccgcttgc gaaaatgcac     1980
   ggtgcgtttt caacgaacgt cgtagcaagc aaagaacttc aacagccagg cagtgcacga     2040
   agcacgcgac atcttgaaat tgaacttcca aaagaagctt cttatcaaga aggagatcat     2100
   ttaggtgtta ttcctcgcaa ctatgaagga atagtaaacc gtgtaacagc aaggttcggc     2160
   ctagatgcat cacagcaaat ccgtctggaa gcagaagaag aaaaattagc tcatttgcca     2220
   ctcgctaaaa cagtatccgt agaagagctt ctgcaatacg tggagcttca agatcctgtt     2280
   acgcgcacgc agcttcgcgc aatggctgct aaaacggtct gcccgccgca taaagtagag     2340
   cttgaagcct tgcttgaaaa gcaagcctac aaagaacaag tgctggcaaa acgtttaaca 2400
   atgcttgaac tgcttgaaaa atacccggcg tgtgaaatga aattcagcga atttatcgcc 2460
   cttctgccaa gcatacgccc gcgctattac tcgatttctt catcacctcg tgtcgatgaa 2520
   aaacaagcaa gcatcacggt cagcgttgtc tcaggagaag cgtggagcgg atatggagaa 2580
   tataaaggaa ttgcgtcgaa ctatcttgcc gagctgcaag aaggagatac gattacgtgc 2640
   tttatttcca caccgcagtc agaatttacg ctgccaaaag accctgaaac gccgcttatc 2700
   atggtcggac cgggaacagg cgtcgcgccg tttagaggct ttgtgcaggc gcgcaaacag 2760
   ctaaaagaac aaggacagtc acttggagaa gcacatttat acttcggctg ccgttcacct 2820
   catgaagact atctgtatca agaagagctt gaaaacgccc aaagcgaagg catcattacg 2880
   cttcataccg ctttttctcg catgccaaat cagccgaaaa catacgttca gcacgtaatg 2940
   gaacaagacg gcaagaaatt gattgaactt cttgatcaag gagcgcactt ctatatttgc 3000
   ggagacggaa gccaaatggc acctgccgtt gaagcaacgc ttatgaaaag ctatgctgac 3060
   gttcaccaag tgagtgaagc agacgctcgc ttatggctgc agcagctaga agaaaaaggc 3120
   cgatacgcaa aagacgtgtg ggctgggtaa 3150
<210> 6
   <211> 1049
   <212> PRT
   <213> Bacillus megaterium
<400> 6
<210> 7
   <211> 37
   <212> DNA
   <213> artificial sequence
<220>
   <223> Forward primer for amplification of phaJ4 gene (restriction site : NheI)
<400> 7
   ccggaagcta gcatgccatt cgtacccgta gcagcgc 37
<210> 8
   <211> 37
   <212> DNA
   <213> artificial sequence
<220>
   <223> Reverse primer for amplification of PHAJ4 gene (restriction site : BamHI)
<400> 8
   ccggaaggat cctcagacga agcagaggct gagggtc 37
<210> 9
   <211> 39
   <212> DNA
   <213> artificial sequence
<220>
   <223> Forward primer for amplification of PHA C2 gene (restriction site : EcoRI)
<400> 9
   ccggaagaat tcatgcgaga aaagcaggaa tcgggtagc 39
<210> 10
   <211> 37
   <212> DNA
   <213> artificial sequence
<220>
   <223> Reverse primer for amplification of PHA C2 gene (restriction site : HindIII)
<400> 10
   ccggaattcg aatcagcgta tatgcacgta ggtgccc 37
<210> 11
   <211> 1176
   <212> DNA
   <213> Pseudomonas aeruginosa
<400> 11
   atgagcctga atccaagaga cgtggtgatt gtcgacttcg gtcgcacgcc gatgggccgc      60
   tccaagggtg gcatgcaccg caatacccgc gccgaagaca tgtcggcgca cctgatcagc     120
   aagctgctgg aacgcaacga caaggtcgac ccgaaagaag tcgaggacgt gatctggggc     180
   tgcgtcaacc agaccctcga gcagggctgg aacatcgccc gcatggcctc gctgatgacc     240
   cagatcccgc acacctccgc ggcgcagacc gtcagccgcc tgtgcggctc gtcgatgagc     300
   gcgctgcaca ccgccgccca ggcgatcatg accggcaacg gtgatgtatt cgtggtcggt     360
   ggcgtggagc acatgggcca cgtcagcatg atgcatggcg tcgaccccaa cccgcacctg     420
   tccttgcatg ccgccaaggc ttccgggatg atgggcctga ccgcggaaat gctcggcaag     480
   atgcacggca tcacccgtga gcagcaggac ctgttcggcc tgcgttcgca ccagctggcg     540
   cacaaggcca cggtcgaagg caagttcaag gacgaaatca tcccgatgca gggctatgac     600
   gagaacggct tcctgaaggt gttcgatttc gacgaaacca ttcgcccgga aaccactctc      660
   gaaggcctgg cgtcgctgaa gccggcgttc aacccgaaag gcggtaccgt cactgccggt      720
   acctcgtcgc agatcaccga cggtgcgtcg tgcatggtcg tcatgtccgg ccagcgcgcc      780
   atggacctgg gtatccagcc attggcggtc atccgttcca tggcggtggc tggtgtcgac      840
   ccggcgatca tgggctacgg cccggtgcca tcgacccaga aagccctcaa gcgtgctggc      900
   ctgaccatgg ccgacatcga cttcatcgag ctcaacgaag ccttcgctgc acaggccctg      960
   ccagtgctga aagacttgaa agtactcgac aagatggatg agaaggttaa cctgcacggc     1020
   ggcgccattg ccttgggcca cccgttcggt tgctccgggg cacggatttc cggcaccctg     1080
   ctcaatgtca tgaagcagaa tggcggtacc ctgggggtgg cgaccatgtg cgtcggcctc     1140
   ggccaaggta tcaccaccgt cttcgagcgc atctaa                               1176
<210> 12
   <211> 1680
   <212> DNA
   <213> Pseudomonas aeruginosa
<400> 12
   atgagtcaga agaacaataa cgagcttccc aagcaagccg cggaaaacac gctgaacctg      60
   aatccggtga tcggcatccg gggcaaggac ctgctcacct ccgcgcgcat ggtcctgctc     120
   caggcggtgc gccagccgct gcacagcgcc aggcacgtgg cgcatttcag cctggagctg     180
   aagaacgtcc tgctcggcca gtcggagcta cgcccaggcg atgacgaccg acgcttttcc     240
   gatccggcct ggagccagaa tccactgtac aagcgctaca tgcagaccta cctggcctgg     300
   cgcaaggagc tgcacagctg gatcagccac agcgacctgt cgccgcagga catcagtcgt     360
   ggccagttcg tcatcaacct gctgaccgag gcgatgtcgc cgaccaacag cctgagcaac     420
   ccggcggcgg tcaagcgctt cttcgagacc ggcggcaaga gcctgctgga cggcctcggc     480
   cacctggcca aggacctggt gaacaacggc gggatgccga gccaggtgga catggacgcc     540
   ttcgaggtgg gcaagaacct ggccaccacc gagggcgccg tggtgttccg caacgacgtg     600
   ctggaactga tccagtaccg gccgatcacc gagtcggtgc acgaacgccc gctgctggtg     660
   gtgccgccgc agatcaacaa gttctacgtc ttcgacctgt cgccggacaa gagcctggcg     720
   cgcttctgcc tgcgcaacgg cgtgcagacc ttcatcgtca gttggcgcaa cccgaccaag     780
   tcgcagcgcg aatggggcct gaccacctat atcgaggcgc tcaaggaggc catcgaggta     840
   gtcctgtcga tcaccggcag caaggacctc aacctcctcg gcgcctgctc cggcgggatc     900
   accaccgcga ccctggtcgg ccactacgtg gccagcggcg agaagaaggt caacgccttc     960
   acccaactgg tcagcgtgct cgacttcgaa ctgaataccc aggtcgcgct gttcgccgac    1020
   gagaagactc tggaggccgc caagcgtcgt tcctaccagt ccggcgtgct ggagggcaag    1080
   gacatggcca aggtgttcgc ctggatgcgc cccaacgacc tgatctggaa ctactgggtc    1140
   aacaactacc tgctcggcaa ccagccgccg gcgttcgaca tcctctactg gaacaacgac    1200
   accacgcgcc tgcccgccgc gctgcacggc gagttcgtcg aactgttcaa gagcaacccg    1260
   ctgaaccgcc ccggcgccct ggaggtctcc ggcacgccca tcgacctgaa gcaggtgact    1320
   tgcgacttct actgtgtcgc cggtctgaac gaccacatca ccccctggga gtcgtgctac    1380
   aagtcggcca ggctgctggg tggcaagtgc gagttcatcc tctccaacag cggtcacatc    1440
   cagagcatcc tcaacccacc gggcaacccc aaggcacgct tcatgaccaa tccggaactg    1500
   cccgccgagc ccaaggcctg gctggaacag gccggcaagc acgccgactc gtggtggttg    1560
   cactggcagc aatggctggc cgaacgctcc ggcaagaccc gcaaggcgcc cgccagcctg    1620
   ggcaacaaga cctatccggc cggcgaagcc gcgcccggaa cctacgtgca tgaacgatga    1680
<210> 13
   <211> 37
   <212> DNA
   <213> artificial sequence
<220>
   <223> Forward primer for amplification of FadA gene (restriction site : XbaI)
<400> 13
   ggccaatcta gaatgagcct gaatccaaga gacgtgg 37
<210> 14
   <211> 32
   <212> DNA
   <213> artificial sequence
<220>
   <223> Reverse primer for amplification of FadA gene (restriction site : HindIII)
<400> 14
   ggccaagctt tagatgcgct cgaagacggt gg 32
<210> 15
   <211> 41
   <212> DNA
   <213> artificial sequence
<220>
   <223> Forward primer for amplification of PHA C1 gene (restriction site : XbaI)
<400> 15
   ggccaatcta gaatgagtca gaagaacaat aacgagcttc c 41
<210> 16
   <211> 36
   <212> DNA
   <213> artificial sequence
<220>
   <223> Reverse primer for amplification of PHA C1 gene (restriction site : HindIII)
<400> 16
   ggccaagctt tcatcgttca tgcacgtagg ttccgg 36
<210> 17
   <211> 40
   <212> DNA
   <213> artificial sequence
<220>
   <223> Forward primer for amplification of CYP102A1 gene (restriction site : NcoI)
<400> 17
   ggccaaccat ggccatgaca attaaagaaa tgcctcagcc 40
<210> 18
   <211> 35
   <212> DNA
   <213> artificial sequence
<220>
   <223> Reverse primer for amplification of CYP102A1 gene (restriction site : BamHI)
<400> 18
   ggccaaggat ccttacccag cccacacgtc ttttg 35